Europäisches Patentamt

European Patent Office

Office européen des brevets

(11) Publication number: **0 174 803 B1**

(12) **EUROPEAN PATENT SPECIFICATION**

(45) Date of publication of patent specification: 21.08.91  (51) Int. Cl.⁵: **A61L 15/16, A61F 13/02**

(21) Application number: 85306297.4

(22) Date of filing: 05.09.85

(54) Adhesive dressings.

(30) Priority: 06.09.84 GB 8422492

(43) Date of publication of application:
19.03.86 Bulletin 86/12

(45) Publication of the grant of the patent:
21.08.91 Bulletin 91/34

(84) Designated Contracting States:
AT BE CH DE FR GB IT LI NL SE

(56) References cited:
EP-A- 0 081 990      EP-A- 0 106 440
EP-A- 0 164 319      FR-A- 2 542 201
GB-A- 1 280 631      GB-A- 2 093 703

(73) Proprietor: SMITH & NEPHEW plc
2 Temple Place Victoria Embankment
London WC2R 3BP(GB)

(72) Inventor: Pratt, William Franklin
3 Birchwood Avenue
Hull North Humberside(GB)

(74) Representative: Cole, William Gwyn
Corporate Patents Department Smith and
Nephew Research Limited Gilston Park
Harlow Essex CM20 2RQ(GB)

Rank Xerox (UK) Business Services

## Description

The present invention relates to adhesive dressings for the treatment of wounds. In particular it relates to an adhesive film dressing which comprises a layer of foam bonded to the non-wound contacting surface of a film which is adhesive coated on the other wound contacting surface and to the methods of forming such dressings.

Adhesive dressings for the treatment of wounds including burns, skin abrasions, surgical incisions and the like and which are moisture vapour permeable have been described in for example British Patent No. 1280631, United Kingdom Patent Applications Nos. 2120104, 2128479 and 2131299, European Patent Applications Nos. 51935, 66899, 81987, 81988, 81898, 81990, 117632 and 120570 and United States Patents Nos. 4413621, 4374520, 4372303, RE 31886 and RE 31887.

Typically the patents and applications describe dressings which have a moisture vapour transmission rate of greater than $300gm^{-2}$ $24hr^{-1}$ at $37°C$ at 100% to 10% relative humidity difference and which when present over a wound prevent bacteria entering the wound environment. The dressings generally comprise a thin flexible film coated with a layer of adhesive on one side. The adhesive coated films because they are thin and flexible have a tendency to crease when being applied to a wound. This causes the film to adhere strongly to itself so spoiling the dressing. Many of the dressings described in the patents listed herein above therefore include in addition to the thin flexible film a handling means which seeks to prevent the flexible film from creasing. Suitable handling means are typically stiff films, frames or handles which may be reversibly adhered to the non-adhesive coated side of the dressing. After the dressing has been successfully applied the handling means are removed. The handling means are generally stiffer than the flexible film and indeed may be rigid in some cases and therefore they will not readily conform to the body when the dressing is in place so they have to be removed. The removal step represents an extra manoeuvre when applying the dressing and may result in the applied dressing being disturbed particularly at its edges which may cause it to be lost from the wound site.

We have found that by using a layer of foam bonded to the non-adhesive coated side of an adhesive dressing, we have obtained a dressing in which the foam acts has a handling means to facilitate application yet does not have to be removed from the flexible film so disturbing the dressing. The foam does not deleteriously affect the flexibility or conformability of the dressing or interfere deleteriously with the moisture vapour transmission rate. The foam provides a cushioned dressing without the necessity of having an absorbent or foam pad on the adhesive side of the dressing. The dressing when dry is opaque and is cosmetically acceptable. However inspection of the wound may be carried out by the simple expedient of wetting the foam whereby the dressing becomes sufficiently translucent to allow visual inspection of the wound beneath the dressing, on drying the dressing become opaque once more. As mentioned herein before the foam bonded to the thin flexible adhesive coated film makes the dressing easy to apply and the dressings of the invention may be used to advantage by untrained personnel as first aid dressings, as well as by trained personnel in the hospital environment.

Accordingly, the present invention provides a bacteria proof adhesive wound dressing having a moisture vapour transmission rate greater than $300gm^{-2}$ $24hrs^{-1}$ at $37°C$ at 100% to 10% relative humidity which dressing comprises a film which is coated on its wound contacting surface by an adhesive layer characterised in that the film has bonded to its non-wound contacting surface a layer of foam.

Where reference is made to moisture vapour transmission rate, it is intended that such measurements are carried out by the Payne cup method which is hereinafter described.

Suitable examples for the foam layer of the dressing include foams made from polyurethanes, styrene butadiene rubber, polyvinyl chloride, polyethylene and latex. These foams may aptly be either open or closed cell or reticulated. Preferred foams are reticulated.

The layer of foam may also be skinned, either as a result of its manufacture, or as a result of a separate process. Preferred foams are made from polyurethanes such as polyester polyurethanes.

The thickness of the foam employed in the dressing of this invention depends on the conformability, elasticity and moisture vapour transmission rate of the foam. Suitably the foam will be from 0.2mm to 15mm thick and more suitably will be from 0.2mm to 5mm, and preferably 0.5mm to 5mm thick for example 0.5mm, 1mm, 2mm, 3mm, 4mm or 5mm. The moisture vapour transmission rate of the selected thickness of foam will be greater than $300gm^{-2}$ $24hr^{-1}$ and preferably will be greater than $500gm^{-2}$ $24hr^{-1}$, at $37°C$ and at 100% td 10% relative humidity.

Aptly the layer of foam may be bonded to the film layer by adhesives. Suitable adhesives include pressure sensitive adhesives, contact adhesives and hot melt pressure sensitive adhesives. Suitable pressure sensitive adhesives are described in British Patent No. 1280631 and No. 2,070,631.

Favoured pressure sensitive adhesives include acrylate ester copolymer adhesives and polyvinyl ether adhesives.

Preferably the foam layer may be bonded to the film layer by flame bonding.

Suitable examples for the film layer of the dressings are described in British Patent Specification No. 1280631, as backing materials. Preferred polymers for forming the film are polyurethanes such as those known as Estane (Registered Trade mark of B.F. Goodrich Ltd.). Suitable Estanes include Estane 5702, 5701, 5714F and 580201.

Other suitable examples for the film layer of the dressing include materials which are elastic and moisture vapour permeable. Such materials include polyetherester block copolymers such as Hytrel 4056.

Yet other preferred polymers for forming the film are polyether polyamide block copolymers such as Pebax (Registered Trade Mark). Suitable Pebax include Pebax 2533 SN 00.

Alternatively the film layer of the dressing may comprise a microporous material such as a microporous polyvinyl chloride film, for example that known as Porvic (Trade Mark). A suitable film is a film $150\mu$m thick.

A second apt film is formed from a polymer blend film. One component of the film comprises the continuous phase in which the second component of the blend is dispersed as discrete particles. By stretching the extruded film voids are formed in the film which do not pass from one side of the film to the other but the film has a suitable moisture vapour transmission rate. Suitable films are described in European Patent Applications Nos. 46071, 72258 and 141952.

The thickness of the film employed in the dressing of this invention will be in the range of 10 to $60\mu$m, suitably 12 to $50\mu$m, more suitably 15 to $40\mu$m and preferably 20 to $35\mu$m. The film will be chosen so that its moisture vapour transmission rate will be greater than 300gm$^{-2}$ 24hr$^{-1}$ and preferably will be greater than 500gm$^{-2}$ 24hr$^{-1}$ at $37°$C and at 100% to 10% relative humidity.

The skin contacting surface of the film of the dressing of this invention is adhesive coated to adhere the dressing to the skin in use. Suitable adhesives must be compatible with the skin, that is they will be hypo-allergenic. Suitable adhesives will be synthetic polymers or mixtures thereof. Such adhesives may be selected from those described in British Patent No. 1280631 and No. 2070631. Preferred adhesives are those which have a moisture vapour transmission rate such that the adhesive together with the film and foam layer has a moisture vapour transmission rate of greater than 300gm$^{-2}$ 24hr$^{-1}$ more suitably the rate will be greater than 500gm$^{-2}$ 24hr$^{-1}$ and preferably greater than 700gm$^{-2}$ 24hr$^{-1}$. Suitable adhesives are those formed from polyacrylate ester copolymers or polyvinyl ethers.

Normally the adhesive will be applied to the film in the form of a continuous layer. However, it is envisaged that the adhesive could be applied to form a discontinuous or pattern spread layer. If desired, the adhesive may incorporate an antibacterial agent such as a chlorhexidine salt.

The size of the dressings of the present invention will vary depending upon the size of wound to which they are applied but will typically have the size of conventional first aid dressings that is they will be generally rectangular in shape with rounded ends or corners. Conventionally the sizes range from 2 x 2cm to 5 x 5cm for square shaped dressings, 2 x 3cm to 5 x 8cm for rectangular first aid dressings and 6 x 8cm to 37.5 x 12cm for hospital use dressings. It is also envisaged that the dressings may be circular or in the form of a long strip or a roll from which individual dressings may be cut as required.

The adhesive surface of the dressings of the present invention must be covered by a protector which may comprise any material known to those skilled in the art for this purpose. Typically the protector is a transparent or translucent material which includes polyvinyl chloride or siliconised paper, such as siliconised glassine paper or other siliconised polymers such as high or low density polyethylene which have been surface treated.

The dressing of this invention may be made by taking an adhesive coated film, the adhesive coated surface of which is protected by a release paper, and flame bonding the foam layer to the non adhesive coated surface of the film. The complete foam, film adhesive dressing may then be cut into the required sizes. The individual dressings may then be sealed into suitable packaging. The dressings contained within the sealed packages may then be sterilised by a suitable method. Suitable methods of sterilisation include $\gamma$-irradiation and ethylene oxide.

The dressing of this invention may also be made by spreading a solution of the material used for the film directly onto a layer of the foam. The adhesive layer may then be coated onto the wound contacting surface of the film that is opposite the film surface bonded to the foam ie. the non-wound contacting surface.

In a further alternative process the dressing of this invention may also be made by casting a solution of polymer forming the film layer onto a release paper and before the solvent has evaporated completely and the film is still tacky, the foam layer is placed onto the wet surface of the film to form the bond between the foam and the film. The foam-film laminate may then be transfer coated onto a precast adhesive layer.

In use, a sterile dressing of the present invention is removed from its bacteria proof pack and the protector removed. The dressing is placed with the adhesive side against the skin and the dressing is adhered to the area surrounding the wound.

The dressings of the present invention may be used to treat a wound on an animal body by adhering a dressing to the area surrounding the wound.

Determination of moisture vapour transmission rate

Discs of material under test are clamped over Payne Permeability Cups (flanged metal cups) using sealing rings and screw clamps. The exposed surface area of the test sample is 10cm². Each cup contains approximately 10mls of distilled water.

After weighing, the cups are placed in a fan assisted electric oven which is maintained at 37 ± 1°C. The relative humidity within the oven is maintained at approximately 10% by placing 1Kg of anhydrous 3-8 mesh calcium chloride on the floor of the oven.

The cups are removed after 24 hours, allowed to cool for 20 minutes and reweighed. The moisture vapour transmission rate of the test material is calculated from the weight loss and expressed in units of grams of weight per square metre per 24 hours.

Example 1

A polyurethane film was cast onto a silicone release paper at a weight of 30 gsm using a polyurethane syrup comprising 100 parts of Estane 5714F (available from B.F. Goodrich Ltd.), 5 parts of Gasil 23 fine silica (available from Crossfield Chemical Ltd.), 240 parts of tetrahydrofuran and 160 parts acetone. The resultant film was then coated on one face ie. the wound contacting surface with a suitable pressure sensitive adhesive at a mass weight of 30 gsm. The adhesive face of the film was then placed onto a silicone release paper and the silicone release paper removed from the non-adhesive face of the film.

A 1mm thick layer of reticulated polyester polyurethane foam was then bonded to the non-adhesive face ie. the non-wound contacting surface of the adhesive coated film. The foam layer was bonded to the adhesive coated film by heating one surface of the foam to about 100°C using a gas flame. The heated foam and the adhesive coated film were then laminated together by passing between the nip of a pair of rollers. The heated face of the foam was bonded to the non-adhesive face of the adhesive coated film. The resultant foam film adhesive dressing was then cut into the appropriate sized individual dressings.

Example 2

A polyether polyamide copolymer was extruded in the conventional manner using a melt temperature of approximately 185°C. The resultant film thickness was approximately 30 microns. This film was then coated on one face with a suitable pressure sensitive adhesive at a mass weight of 35 gsm. The adhesive face of the film was then placed onto a silicone release paper.

A 2mm thick layer of reticulated polyethylene foam was then bonded to the non-adhesive face fo the adhesive coated film. The foam layer was bonded to the adhesive coated film by heating one surface of the foam to 100°C using a gas flame. The heated foam and the adhesive coated film were then laminated together so that the heated face of the foam was bonded to the non-adhesive coated face of the adhesive coated film. The resultant foam, film, dressing was then cut into the appropriate sized individual dressings.

Example 3

A film of a polyether polyester elastomer, (Hytrel 4065, available from E.I. Du Pont de Nemours & Co) was prepared by extrusion of the polymer onto a siliconised release surface at a melt temperature of 204°C. The film was then passed between a pair of nip rollers to give a film of thickness 37.5 $\mu$m.

This film was then coated on one surface with an adhesive layer formed by casting an acetone solution containing an acrylate ester copolymer comprising a copolymer formed by polymerising 47 parts by weight of 2-ethyl hexyl acrylate, 47 parts by weight n-butyl acrylate and 6 parts by weight acrylic acid onto a siliconised release paper.

A 2mm thick layer of reticulated polyester polyurethane foam was then bonded to the non-adhesive face of the adhesive coated film in a similar manner to that described in Example 1. The resultant laminate was then cut into appropriate sized dressings and each was packed into a bacteria proof pack and sealed. The pack may be sterilised by gamma-irradiation.

In use the sterile dressing is removed from the pack, the protector layer removed and the dressing adhered to the area surrounding the wound.

Example 4

A film of thermoplastic polymer is formed by extruding a blend of ethylene-vinyl acetate copolymer, 90 parts by weight and high impact polystyrene, 10 part by weight. The film has a thickness of 75$\mu$m. The film was then stretched in a direction transverse to the machine direction to a draw ratio of 4.1 at ambient room temperature 18 to 22°C and the drawn film allowed to contract to 2.5:1 so as to form a voided film which has a moisture vapour transmission rate of 1800 gm$^{-2}$ 24hr$^{-1}$ at 37°C at 100% to 10% relative humidity difference.

This film is coated on one surface with a vinyl ethyl ether pressure sensitive adhesive cast onto a release paper. The non-adhesive side of the film is adhered to a polyurethane foam using a vinyl ethyl ether pressure sensitive adhesive.

The resulting laminate is cut into suitably sized pieces and packaged in a bacteria proof pack.

## Claims

1. A bacteria proof adhesive wound dressing having a moisture vapour transmission rate greater than 300 gm$^{-2}$ 24hrs$^{-1}$ at 37°C at 100% to 10% relative humidity difference which dressing comprises a film which is coated on its wound contacting surface by an adhesive layer characterised in that the film has bonded to its non wound contacting surface a layer of foam.

2. A wound dressing as claimed in claim 1 in which the foam is from 0.5 to 15mm thick.

3. A wound dressing as claimed in either of claims 1 or 2 in which the foam is a reticulated foam.

4. A wound dressing as claimed in any of claims 1 to 3 in which the foam is formed from a polyurethane.

5. A wound dressing as claimed in any of claims 1 to 4 in which the layer of foam is bonded to the non-wound contacting surface of the film by means of a pressure sensitive adhesive.

6. A wound dressing as claimed in any of claims 1 to 4 in which the layer of foam is bonded to the non-wound contacting surface of the film by means of flame bonding.

7. A wound dressing as claimed in any of claims 1 to 6 in which the film has a thickness of from 10 to 60 microns and a moisture vapour transmission rate of greater than 300 gm$^{-2}$ 24 hr$^{-1}$ at 37°C and at 100% to 10% relative humidity difference.

8. A wound dressing as claimed in claim 7 in which the film is a polyurethane.

9. A wound dressing as claimed in claim 7 in which the film is a polyetherester block copolymer.

10. A wound dressing as claimed in any one of claims 1 to 9 in which the adhesive layer is continuous and comprises a pressure sensitive adhesive.

11. A wound dressing as claimed in claim 1 in which the adhesive surface of the dressing is covered by a protector.

12. A wound dressing as claimed in claim 11 which is sterile and is packaged in a bacteria proof pack.

## Revendications

1. Pansement pour plaie adhésif étanche aux bactéries ayant un taux de transmission de vapeur d'eau supérieur à 300 g/m²/24 heures à 37°C à une différence d'humidité relative de 100 % à 10 %, lequel pansement comprend un film qui est revêtu sur sa surface en contact avec la plaie d'une couche adhésive, caractérisé en ce qu'une couche de mousse est liée sur la surface du film qui n'est pas en contact avec la plaie.

2. Pansement pour plaie suivant la revendication 1, caractérisé en ce que la mousse a une épaisseur de 0,5 à 15 mm.

3. Pansement pour plaie suivant l'une quelconque des revendications 1 ou 2, caractérisé en ce que la mousse est une mousse réticulée.

4. Pansement pour plaie suivant l'une quelconque des revendications 1 à 3, caractérisé en ce que la mousse est formée à partir d'un polyuréthane.

5. Pansement pour plaie suivant l'une quelconque des revendications 1 à 4, caractérisé en ce que la couche de mousse est fixée à la surface du film qui n'est pas en contact avec la plaie à l'aide d'un adhésif sensible à la pression.

6. Pansement pour plaie suivant l'une quelconque des revendications 1 à 4, caractérisé en ce que la couche de mousse est fixée à la surface du film qui n'est pas en contact avec la plaie par liaison à la flamme.

7. Pansement pour plaie suivant l'une quelconque des revendications 1 à 6, caractérisé en ce que le film a une épaisseur de 10 à 60 μm et un taux de transmission de vapeur d'eau supérieur à 300 g/m²/24 heures à 37° C et à une différence d'humidité relative de 100 % à 10 %.

8. Pansement pour plaie suivant la revendication 7, caractérisé en ce que le film est un polyuréthane.

9. Pansement pour plaie suivant la revendication 7, caractérisé en ce que le film est un copolymère à bloc de polyétherester.

10. Pansement pour plaie suivant l'une quelconque des revendications 1 à 9, caractérisé en ce que la couche adhésive est continue et comprend un adhésif sensible à la pression.

11. Pansement pour plaie suivant la revendication 1, caractérisé en ce que la surface adhésive du pansement est recouverte d'une protection.

12. Pansement pour plaie suivant la revendication 11, caractérisé en ce qu'il est stérile et conditionné dans un emballage étanche aux bactéries.

**Patentansprüche**

1. Für Bakterien undurchlässiges Wund-Heftpflaster mit einer Durchlässigkeit für Wasserdampf von über 300 gm$^{-2}$ 24 h$^{-1}$ bei 37° C und 100 - 10 % relativer Feuchtigkeitsdifferenz, welches einen Film aufweist, der an seiner mit der Wunde in Berührung kommenden Oberfläche mit einem Klebstoff überzogen ist, dadurch gekennzeichnet, daß an dem Film an seiner mit der Wunde nicht in Berührung kommenden Oberfläche eine Schaumstoffschicht angebracht ist.

2. Wundpflaster nach Anspruch 1, bei welchem der Schaumstoff zwischen 0,5 - 15 mm dick ist.

3. Wundpflaster nach Anspruch 1 oder 2, bei welchem der Schaumstoff ein netzförmiger Schaumstoff ist.

4. Wundpflaster nach einem der Ansprüche 1 - 3, bei welchem der Schaumstoff aus einem Polyurethan gebildet ist.

5. Wundpflaster nach einem der Ansprüche 1 - 4, bei welchem die Schaumstoffschicht mit der mit der Wunde nicht in Berührung kommenden Oberfläche des Films mittels eines druckempfindlichen Klebstoffes verbunden ist.

6. Wundpflaster nach einem der Ansprüche 1 - 4, bei welchem die Schaumstoffschicht mit der mit Wunde nicht in Berührung kommenden Oberfläche des Films mittels Flammlaminieren verbunden ist.

7. Wundpflaster nach einem der Ansprüche 1 - 6, bei welchem der Film eine Dicke von 10 - 60 Mikron und eine Durchlässigkeit für Wasserdampf von mehr als 300 gm$^{-2}$ 24 h$^{-1}$ bei 37° C und 100 - 10 % relativer Feuchtigkeitsdifferenz aufweist.

8. Wundpflaster nach Anspruch 7, in welchem der Film ein Polyurethan ist.

9. Wundpflaster nach Anspruch 7, in welchem der Film ein Polyätherester Blockcopolymer ist.

10. Wundpflaster nach einem der Ansprüche 1 - 9, bei welchem der Klebstoffüberzug kontinuierlich ist und einen druckempfindlichen Klebstoff aufweist.

11. Wundpflaster nach Anspruch 1, bei welchem die Klebstoffoberfläche des Pflasters durch eine Schutzfolie abgedeckt ist.

12. Wundpflaster nach Anspruch 11, welches steril und in einer für Bakterien undurchlässigen Packung verpackt ist.